Europäisches Patentamt

European Patent Office

Office européen des brevets

(11) Publication number: **0 212 786**
**A1**

## (12) EUROPEAN PATENT APPLICATION

(21) Application number: 86303983.0

(51) Int. Cl.⁴: **A61B 17/36**

(22) Date of filing: 27.05.86

(30) Priority: 02.08.85 US 762052

(43) Date of publication of application:
04.03.87 Bulletin 87/10

(84) Designated Contracting States:
DE FR GB IT NL

(71) Applicant: **C.R. Bard, Inc.**
**731 Central Avenue**
**Murray Hill, New Jersey 07974(US)**

(72) Inventor: **Sinofsky, Edward**
**Apartment 8 South Washington Street**
**Reading, Massachusetts(US)**

(74) Representative: **Woodward, John Calvin et al**
**VENNER SHIPLEY & CO. 368 City Road**
**London EC1V 2QA(GB)**

(54) **Laser/optical fiber safety apparatus and method.**

(57) A safety apparatus which prevents injury or damage in a laser/optical fiber system in which laser power is transmitted from a source down an optical fiber to irradiate a remote workpiece. The safety apparatus detects overheating of the optical fiber - (caused by fiber end contamination or fiber breakage) by detecting infrared thermal radiation which is generated when the laser energy is absorbed by the fiber.

The infrared thermal energy propagates through the fiber (216) back towards the laser source (200) and is reflected by a mirror (208) out of the laser beam path (206) onto a suitable detector (232). When the output of the detector rises above an adjustable threshold voltage, a shutter mechanism - (204) is operated which interrupts the laser beam. The apparatus may be used with many types of lasers, including infrared lasers, by using an appropriate filter (228) to block the laser energy and prevent it from reaching the detector.

FIG. 2

Xerox Copy Centre

## LASER/OPTICAL FIBER SAFETY APPARATUS AND METHOD

This invention relates to safety apparatus for use with optical fiber systems that transmit high-power laser energy, and more particularly to apparatus which prevents heat damage to the fiber, a catheter containing the fiber and its surrounding environment caused by fiber breakage or contamination.

Energy developed by high-power lasers has been used for many years in industry for material cutting and removal. More recently, lasers with somewhat less power have also been used for medical surgical purposes for cutting and removal of body tissues.

In both the industrial and medical environments it is often convenient to transmit the laser energy down an optical fiber to the desired work area. The use of an optical fiber is particularly convenient for surgical uses if the optical fiber can be made small enough to be inserted inside medical catheters. Such an arrangement allows percutaneous surgery where only a small incision must be made to insert the catheter.

One problem which has been encountered with the use of laser cutting or material removal systems is heat damage to the fiber and its surrounding environment caused by fiber-end contamination or fiber breakage. More particularly, when high-power laser energy is transmitted through an optical fiber, problems often occur due to breaks in the fiber core and contamination of the distal fiber end by materials evaporated from the work area (in the case of industrial applications) or by blood or tissue (in the case of medical applications). When either of the foregoing events occurs the laser energy becomes trapped in the fiber instead of being transmitted through the fiber and heat caused by dissipation of the laser energy within the fiber can destroy the fiber by melting or vaporization or can damage the fiber's surroundings.

Fiber overheating is a particular problem in medical applications utilizing a catheter to contain the fiber because heat created by the dissipation of the laser energy within a damaged or contaminated fiber can scald the catheter pathway back to the point of entry into the body or melt the catheter in which the fiber is enclosed. In either case the injury to the patient can be severe.

Accordingly, several prior art approaches have been developed to prevent optical fiber damage due to overheating. One such approach is to divert a portion of the laser energy emitted from the optical fiber to a suitable detector. If the amount of power emitted by the fiber falls outside of predetermined bounds, the laser source is shut down. This approach suffers from two major disadvantages:

the overall output power is reduced since part of the power must be diverted for monitoring purposes and a beam splitter must be positioned at the output end of the fiber in order to branch the laser light to the detector.

Other prior art systems, such as that disclosed in U.S. patent 4,423,726, detect heat damage caused by contamination at the end of the fiber. In the presence of such contamination, the output end of the fiber may become so overheated by the laser energy that it partially evaporates. Evaporated substances then condense on objects such as lenses which are in the vicinity of the fiber end. Various arrangements are used to detect the presence of these evaporated substances including photodetectors which are sensitive to the changes in the transmittance or reflectance of the objects caused by the evaporated substances. This prior art approach suffers from the disadvantage that it cannot detect overheating until the temperature increases to the point where evaporation of the fiber occurs. In addition apparatus must be present at the output end of the fiber to monitor the output energy.

Another prior art approach is exemplified in U.S. patents 4,385,832; 4,320,968 and 4,321,707 and uses reflected laser light to detect a fiber break or fiber-end contamination. In such a system, light reflected from the fiber end traveling back toward the laser source is separated from the main laser beam and monitored. Contamination at the end of the fiber or a break in the fiber causes a change in the amount of laser light which is reflected back through the fiber. When the reflected light changes outside of predetermined bounds, an alarm is sounded or appropriate action is taken. Although this system does not require apparatus at the output end of the fiber it cannot detect certain types of fiber damage which do not change the fiber reflectivity.

Still other prior art approaches use sensors located along the fiber to detect overheating caused by a break. For example, U.S. patent number 4,476,512 discloses the use of a plurality of thermistors applied to the fiber optic cable at selected spots along its length. A circuit monitors the output of the thermistors to detect abnormal increases or decreases in cable temperature due to breakage. In a similar arrangement, U.S. patent 4,311,142 utilizes a conducting wire which runs along a fiber core path. A break in the fiber causes the wire to melt or causes a variance in the wire resistance due to the raised temperature. A detector connected to the end of the wire monitors the wire's resistance and turns off the laser if the resistance changes abnormally. These latter systems may not be able to detect fiber-end contamination unless sensors are located at the fiber end.

Accordingly, it is an object of the present invention to prevent heat damage of an optical fiber due to fiber damage or contamination.

It is another object of the present invention to provide a heat damage protection system which requires no sensor connections to the optical fiber.

It is yet another object of the present invention to provide a heat damage prevention system which can detect fiber heat damage caused by both fiber breakage and fiber-end contamination.

It is still another object of the present invention to provide a heat damage prevention system which can disable the laser energy source before heat damage occurs.

It is further object of the present invention to provide a heat damage prevention system which has an easily adjustable threshold which can be preset to disable the laser energy source when the fiber reaches a predetermined temperature.

It is still a further object of the present invention to provide a heat damage prevention system which is usable with many different types of lasers.

It is another object of the present invention to provide a heat damage prevention system which will also prevent damage to the laser system if the laser is misaligned to the fiber or if the workpiece overheats.

The foregoing problems are solved and the foregoing objects are achieved in one illustrative embodiment of the invention in which infrared thermal radiation traveling back through the fiber is monitored and the laser energy source is disabled if the monitored radiation falls outside of predetermined limits.

More particularly, the fiber which transmits the laser energy to the work area is manufactured from materials which will transmit infrared thermal radiation. During operation of the laser/fiber system, thermal radiation is generated by the fiber itself due to dissipation of the laser energy in the fiber. Some of the thermal energy propagates through the fiber back toward the proximal end of the fiber and the laser energy source. At the proximal fiber end, the infrared thermal energy is reflected by a mirror to an infrared detector. A filter is used to separate the thermal radiation from reflected laser energy so that the detector only responds to the thermal energy.

During normal laser/fiber operation only a small amount of thermal radiation reaches the detector because the optical fiber does not transmit energy with a wavelength longer than a "cutoff" wavelength and, at the normal operation termperatures, the amount of thermal radiation with wavelengths shorter than the cutoff wavelength is small. However, under a condition in which a break in the fiber occurs or the fiber end becomes contaminated, the temperature of the fiber rises causing increasing amounts of thermal radiation with wavelengths less than the cutoff wavelength to be generated. The increasing amounts of short-wavelength thermal radiation passing through the fiber, in turn, cause the detector signal to increase.

The detector signal is amplified and compared to a threshold signal and used to activate a shutter mechanism if the detector signal rises above the threshold signal. The shutter interrupts the laser beam. The threshold signal can be set to limit the temperature rise of the fiber to a predetermined value. Thus heat damage caused by overheating of the fiber is prevented.

In accordance with one aspect of the invention, the illustrative system also senses and prevents overheating due to misalignment of the laser beam and overheating of the workpiece. More particularly, if the optical fiber is misaligned with the laser beam, the beam will impinge on the end of the fiber connector causing it to overheat. Thermal radiation generated by the heated fiber connector will then be focussed onto the detector and disable the laser if the fiber-end temperature rises above the predetermined threshold. Similarly, if the workpiece overheats (which may, for example, occur during a surgical operation if the laser beam impinges on a solid object such as a bone or calcified atherosclerotic plaque) thermal radiation generated by the overheated workpiece is transmitted through the fiber and operates the overheating control in the same manner as an overheated fiber.

Figure 1 is a sketch of the intensity of thermal radiation generated by an optical fiber versus wavelength for different fiber temperatures.

Figure 2 is a block schematic diagram of the inventive heat damage prevention system.

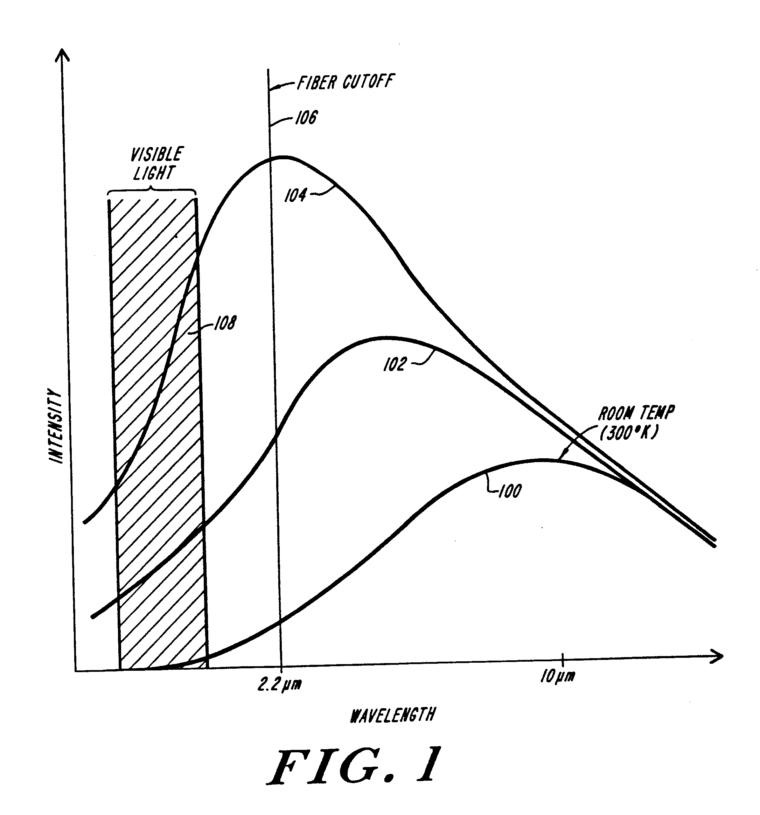

Figure 1 is a sketch of intensity versus wavelength for thermal or "blackbody" radiation generated by an object, such as an optical fiber, at three different temperatures. More particularly, the vertical axis represents intensity increasing in an upward direction. The horizontal axis indicates wavelength (in micrometers) increasing towards the right.

Curve 100 represents the thermal radiation spectrum of an object at room temperature (300° Kelvin). As shown in Figure 1, the thermal radiation emitted by the object with the highest intensity has a wavelength of approximately 10 micrometers. However, significant intensities of thermal radiation are found at wavelengths which are either longer or shorter than the 10 micrometer peak.

The visible light region is shown in Figure 1 as region 108. Radiated energy with a wavelength falling within region 108 can be perceived by the eye as colored light. The eye perceives a red color when energy with the longer wavelengths in the visible region is radiated and a violet color when energy with the shorter wavelengths is radiated. As shown in Figure 1, thermal radiation from an object at room temperature emits virtually no radiation within the visible light band and thus the eye perceives no colors due to thermal radiation.

However, as the temperature of the object increases, the intensity peak of the thermal energy curve moves to shorter wavelengths as exemplified by curves 102 and 104 and more and more radiation appears in the visible light band 108. As the temperature increases, at first only a significant amount of energy is found at the longer wavelengths of the visible light spectrum and, thus, the object appears to be "red hot". However, as the temperature rises higher and higher more significant amounts of energy appears at the shorter visible wavelengths and thus the object becomes "white hot".

More importantly, a typical optical fiber will transmit energy with wavelengths up to a wavelength limit known as the "cut-off" wavelength. The fibers will not transmit energy with wavelengths longer than the cut-off limit. For example, silica fibers which have been specially treated to reduce the hydroxyl-ion content have a cutoff wavelength of 2.2 micrometers (this limit is marked in Figure 1 as line 106).

Thus, referring to Figure 1, it can be seen that, for an object at room temperature, very little thermal radiation is transmitted through the fiber since only the low "tail" portion of the radiation curve lies at wavelengths less than the cutoff line 106. However, as the temperature increases and the radiation wavelengths change as depicted in radiation curves 102 and 104, more significant amounts of energy have wavelengths less than the cut-off wavelength and are transmitted by the fiber.

In accordance with the invention, the amount of energy transmitted by the fiber can be directly related to the temperature of an object generating the thermal radiation even where the object is the fiber itself. Therefore, it can be seen that a detector which detects the intensity of the thermal radiation transmitted through the fiber can be used to monitor the temperature of the fiber.

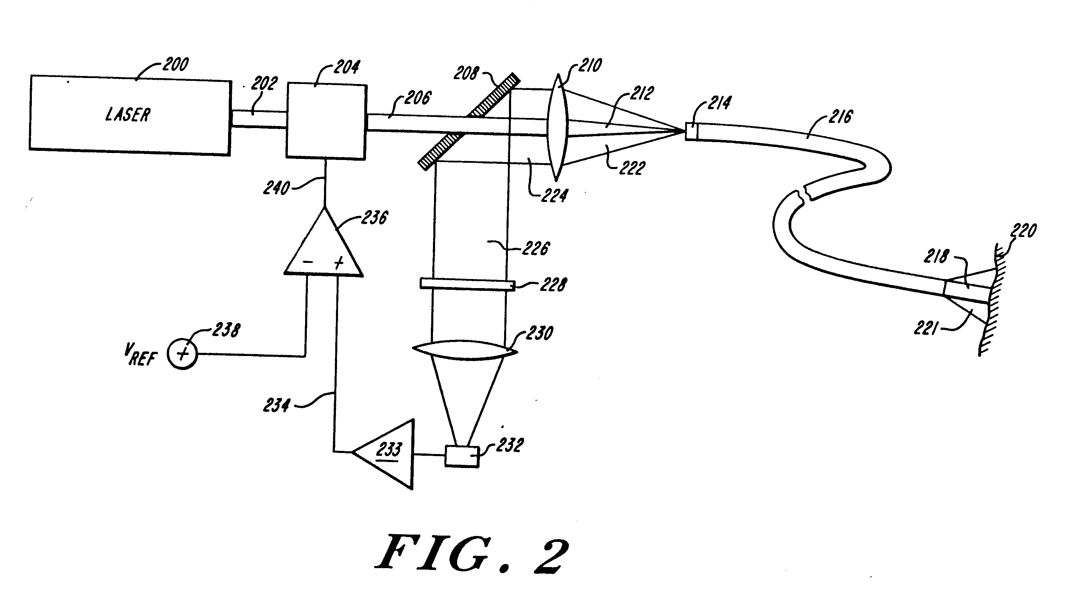

Figure 2 shows an illustrative apparatus which can be used to measure the temperature of the fiber. In Figure 2, laser 200 may illustratively be one of a number of different laser types which produce a beam which is suitable for use for a particular application. For example, laser 200 may be a carbon dioxide laser which is suitable in industrial applications for drilling and cutting purposes.

For medical purposes, laser 200 is more preferably a laser which generates infrared output energy in the wavelength range of 1.4-2.2 microns or an Argon-ion laser. Illustrative lasers which generate an output with a wavelength in this region are Holmium-doped Yttrium-Lithium-Fluoride (YLF); Erbium-doped YLF; Thulium-doped YLF and Holmium, Erbium, and Thulium-doped Yttrium-Aluminum-Garnet. The output wavelength region of 1.4-2.2 microns has certain advantages for surgical applications in that fused-silica optical fibers which are suitable for use within the human body can be used to transmit the laser energy. The use of such lasers for surgical purposes is described in detail in my co-pending U.S. patent application entitled "Infrared Laser Catheter System", filed on July 31, 1985 and assigned Serial No. ........ and assigned to the same assignee as the present invention which application is herein incorporated by reference.

The output beam 202 from laser 200 passes through a shutter 204. Shutter 204 may be a conventional mechanical or optical shutter which is activated by a signal from lead 240. Upon activation, shutter 204 disables laser 200 by interrupting its output beam 202.

Assuming that the shutter is open, beam 202 passes through shutter 204 as beam 206 and impinges on focusing lens 210. Located between focusing lens 210 and shutter 204 is a mirror 208 (which is shown in cross-section in Figure 2). Mirror 208 has a small hole drilled through its center through which beam 206 passes. As shown in Figure 2, mirror 208 is inclined at a 45° angle with respect to the beam axis to reflect the thermal radiation developed by fiber 216, as will be hereinafter described.

Beam 206 is focused by lens 210 to produce focused beam 212 which impinges on the end of optical fiber 216 which is, in turn, held in a conventional fiber optic connector 214. Optical fiber 216 may be contained within a conventional catheter - (not shown) if the laser system is being used for percutaneous laser surgery. At the distal end of fiber 216 the beam 218 exits and is applied to the work surface 220 which may be an industrial workpiece or a surgical site.

Thermal energy developed within fiber 216 travels through the fiber towards both the distal end and the proximal end (and fiber optic connector 216). Because the thermal energy is developed by random atomic motions within the fiber itself, the thermal energy is emitted in random directions and is not focussed into a collimated beam as is the laser energy. Thus, the returning thermal energy spreads out in a cone 222 which is wider than the focussed laser beam 212. This cone is, in turn, focused by focusing lens 210 into a wide beam 224 which strikes the surface of mirror 208 and is reflected as beam 226 to filter 228. A small amount of energy passes through the central hole in mirror 208, but since the diameter of the hole is small relative to the mirror surface, most of the energy is reflected by the mirror.

Filter 228 absorbs all incoming radiation except the infrared thermal radiation which passes through. More particularly, filter 228 is a conventional optical filter which is designed to absorb all radiation with the exception of radiation having a wavelength in the range of 1 to 2.7 micrometers. The radiation which passes through filter is focused by lens 230 onto detector 232. Detector 232 is a conventional photodetector which is sensitive to energy in the wavelength range passed by filter 228.

The output signal from photodetector 232 is amplified by amplifier 233 and provided to the positive input of a comparator 236, via lead 234. Comparator 236 receives a reference voltage developed by reference source 238. Reference source 238 is adjustable and can be used to set the threshold for shutter operation. Comparator 236 produces an output signal on lead 240 which, in turn, controls shutter 204.

In accordance with the invention, laser 200 is operated to irradiate workpiece 220. If fiber 216 breaks or if the end of fiber 216 becomes contaminated with material vaporized from workpiece 220 - (or, in surgical applications, with coagulated blood or tissue), the temperature of the fiber rises because laser energy is absorbed by the fiber. As the temperature rises, the amount of thermal radiation which propagates back through the fiber will increase because the amount of shorter wavelength radiation which can be transmitted by the fiber increases. The increased amount of transmitted radiation, in turn, causes the output signal generated by photodetector 232 to increase in magnitude. When the output of photodetector 232 rises above the threshold voltage generated by reference source 238, shutter 204 is activated and interrupts the laser beam and prevents overheating and thermal damage to the fiber and its surroundings.

## Claims

1. Thermal damage prevention apparatus for use in a laser system having an optical fiber with a proximal end and a distal end and a laser with an output beam focused onto said proximal fiber end, said apparatus being characterised by a detector - (232) sensitive to thermal infrared radiation, said dectector developing an output signal whose magnitude is related to the intensity of said thermal infrared radiation, directing means (208) for directing infrared thermal radiation emerging from said proximal end of said fiber onto said detector, control generating means (236,238) responsive to said detector output signal for generating a control signal when said detector output signal is greater than a predetermined magnitude, and disabling means - (204) responsive to said control signal for disabling said laser output beam.

2. Apparatus according to claim 1 characterised in that the directing means includes filter means (228) for preventing all energy other than thermal radiation from reaching said detector.

3. Apparatus according to claim 1 or claim 2 characterised in that the control signal generating means comprises a reference source (238) for generating a reference signal and comparator means (236) responsive to the reference signal and to the detector output signal for generating said control signal when the detector output signal exceeds the reference signal.

4. Apparatus as claimed in claim 2 or claim 3 wherein the detector is a photodetector (232) and the directing means (208) diverts infrared thermal radiation emerging from the proximal end of the fiber out of the laser beam path, the apparatus being further characterised in that the filter means - (228) is disposed in the diverted radiation path and absorbs all energy except said thermal radiation, focussing means (23), being disposed between the filter means (228) and photodetector (230) for focussing energy passing through the filter means onto the photodetector.

5. Apparatus according to claim 4 characterised in that the means responsive to the detector output signal is a reference source (238) for generating a reference signal and a comparator responsive to said reference signal for generating the control signal when the detector output signal exceeds said reference signal.

6. Apparatus according to any one of the preceding claims characterised in that the disabling means is a mechanical shutter (204) located between the laser and the proximal fiber end which is responsive to the control signal to interrupt the laser output beam.

7. Apparatus according to anyone of claims 2 - 6 characterised in that the filter means (230) only allows energy having a wavelength in the range of 1-2.7 micrometers to pass through it.

8. Apparatus according to any one of the preceding claims characterised in that the diverting means comprises a mirror (208) having a surface reflective to said thermal radiation, the mirror being located between the laser (200) and the proximal fiber end and having a hole through the center thereof through which the laser beam passes.

9. Apparatus according to Claim 8 further characterised by a lens (210) located between the mirror (208) and the proximal end (214) of the fiber, said lens focussing the laser beam onto the proximal end of the fiber.

10. A method for preventing thermal damage in a laser system having an optical fiber with a proximal end and a distal end and a laser with an output beam focused onto said proximal fiber end, said method comprising the steps of:

A) monitoring the intensity of infrared thermal radiation emerging from the proximal end of the fiber, and

B) disabling the laser output beam when said thermal radiation intensity exceeds predetermined limits.

11. A method according to Claim 10 characterised in that step A) comprises the steps of:

A1) diverting the infrared thermal radiation emerging from the proximal end of the fiber out of the laser beam path,

A2) placing a filter in said diverted radiation path to absorb all energy except said thermal radiation, and

A3) directing energy passing through the filter means onto a photodetector,

12. A method according to Claim 11 characterised in that the photodetector has an output signal and step A further comprises the steps of:

A4) comparing the output signal of the photodetector to a predetermined reference signal, and

A5) interrupting the laser beam when the magnitude of the output of the photodetector exceeds the magnitude of said reference signal.

13. A method as claimed in any one of claims 11 -13 characterised by the further step of placing a mirror at said proximal fiber end to divert infrared thermal radiation emerging from said proximal end of the fiber out of said laser beam path.

14. A method as claimed in Claim 13 characterised in that the laser output is disabled by operating a shutter located between the laser and the proximal fiber end to interrupt said laser output beam when the magnitude of the photodetector output signal exceeds the magnitude of the predetermined reference signal.

FIG. 1

LASER

200

202

204

206

208

210

212

214

216

218

220

221

222

224

226

228

230

232

233

234

236

238 $V_{REF}$

240

*FIG. 2*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | GB-A-2 034 883 (ELLIOTT BROTHERS LTD.) <br> * Figure 2; abstract; page 1, line 111 - page 2, line 5 * <br><br> --- | 1,3-5 | A 61 B 17/36 |
| A | US-A-4 236 904 (P.D. LAZAY) <br><br> * Figure 1; column 2, lines 20-23; column 2, line 41 - column 3, line 4; column 4, lines 33-66 * <br><br> --- | 1,2,4, 8,9 | |
| A | PATENTS ABSTRACTS OF JAPAN, vol. 6, no. 133 (P-129)[1011], 20th July 1982; & JP-A-57 57 222 (MEDOSU KENKYUSHO K.K.) 06-04-1982 <br><br> --- | 8,9 | |
| A | PATENTS ABSTRACTS OF JAPAN, vol. 6, no. 175 (P-141)[1053], 9th September 1982; & JP-A-57 93 228 (MEDOSU KENKYUSHO K.K.) 10-06-1982 <br><br> --- | 6 | TECHNICAL FIELDS SEARCHED (Int. Cl.4) <br><br> A 61 B <br> G 01 M <br> H 04 B |
| A | US-A-3 981 592 (D.N. WILLIAMS) <br><br> ----- | | |

The present search report has been drawn up for all claims

| Place of search | Date of completion of the search | Examiner |
|---|---|---|
| THE HAGUE | 06-11-1986 | SEDY, R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82